(19) Europäisches Patentamt
European Patent Office
Office européen des brevets

(11) EP 2 181 998 B1

(12) **EUROPEAN PATENT SPECIFICATION**

(45) Date of publication and mention of the grant of the patent:
**19.09.2012 Bulletin 2012/38**

(51) Int Cl.:
***C07D 491/113*** *(2006.01)* ***A61K 31/5415*** *(2006.01)*
***A61P 31/04*** *(2006.01)*

(21) Application number: **10153291.9**

(22) Date of filing: **24.05.2006**

(54) **New benzothiazinone derivative and its use as antibacterial agent**

Neues Benzothiazinon-Derivat und seine Verwendung als antibakterieller Wirkstoff

Nouveau dérivé de benzothiazinone et son utilisation en tant qu'agent antibactérien

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**05.05.2010 Bulletin 2010/18**

(62) Document number(s) of the earlier application(s) in accordance with Art. 76 EPC:
**06743049.6 / 2 029 583**

(73) Proprietor: **Leibniz-Institut für Naturstoff-Forschung und Infektionsbiologie e.V. -Hans-Knöll-Institut- (HKI)
07745 Jena (DE)**

(72) Inventors:
- **Makarov, Vadim A.
129090 Moskow (RU)**
- **Cole, Stewart T.
CH-1024 Ecublens (CH)**
- **Möllmann, Ute
07749 Jena (DE)**

(74) Representative: **HOFFMANN EITLE
Patent- und Rechtsanwälte
Arabellastrasse 4
81925 München (DE)**

(56) References cited:
**WO-A1-2005/092872 US-A- 3 522 247**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 181 998 B1

## Description

The present invention relates to a novel benzothiazin derivative and its use as antibacterial agent in infectious diseases of mammals (humans and animals) caused by bacteria, especially diseases like tuberculosis (TB) and leprosy caused by *mycobacteria*.

Thiazinone, their derivatives and their use as antibacterial agents, especially against mycobacteria (TB), laid open for public in AR 24 25 67 A1, AU 37 04 400 A1, CA 13 22 551 C1 or EP 0 245 901 B1 for instance.

As known, there is a threadful worldwide increase in tuberculosis infections with *mycobacteria* which developed resistance against the available therapeutics (B.R.Bloom, J.L.Murray, tuberculosis: commentary on a reemergent killer. Science 257, 1992, 1055-1064). Extremely dangerous is the development of multidrug resistant (MDR) *mycobacteria*. These are *mycobacteria*, resistant at least against two of the most active tuberculosis drugs, isoniazid and rifampicin, but also against streptomycin, pyranzinamid and ethambutol. The proportion of MDR-TB in some countries is already more than 20%. Together with the increased number of TB diseases generally, worldwide it causes about 3.000.000 deaths annually.

For the treatment of such diseases, like (TB) or leprosy there is an urgent need for new drugs with new mechanisms of actions, especially to overcome drug resistance and to overcome the known dramatic side effects of the available drugs.

<u>Object of the invention</u>

The present invention aims at the generation of new compounds with activity against *mycobacteria* as potential new tuberculosis drugs to overcome problems concerning resistance and drug intolerance.

<u>Solution of the technical problem</u>

This aim has been solved by providing 2-(2-methyl-1,4-dioxa-8-azaspiro[4.5]dec-8-yl)-8-nitro-6-(trifluoromethyl)-1,3-benzothiazin-4-one and salts thereof.

For the synthesis of the aimed compound we developed our original method of 1,3-benzothiazin-4-one synthesis with usage of dithiocarbamate derivatives as intermediate (method A). The classical method of 1,3-benzothiazin-4-one synthesis with usage of thiocyanate salts (method B) is usable too. Both are presented in the scheme below, wherein $R^1$ is $NO_2$, $R^2$ is trifluoromethyl and $R^5$ and $R^6$ together represent the bivalent radical

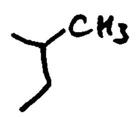

.

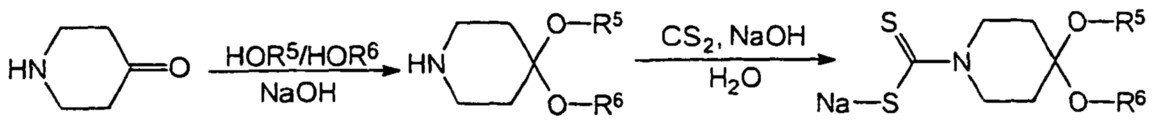


Na-DTC

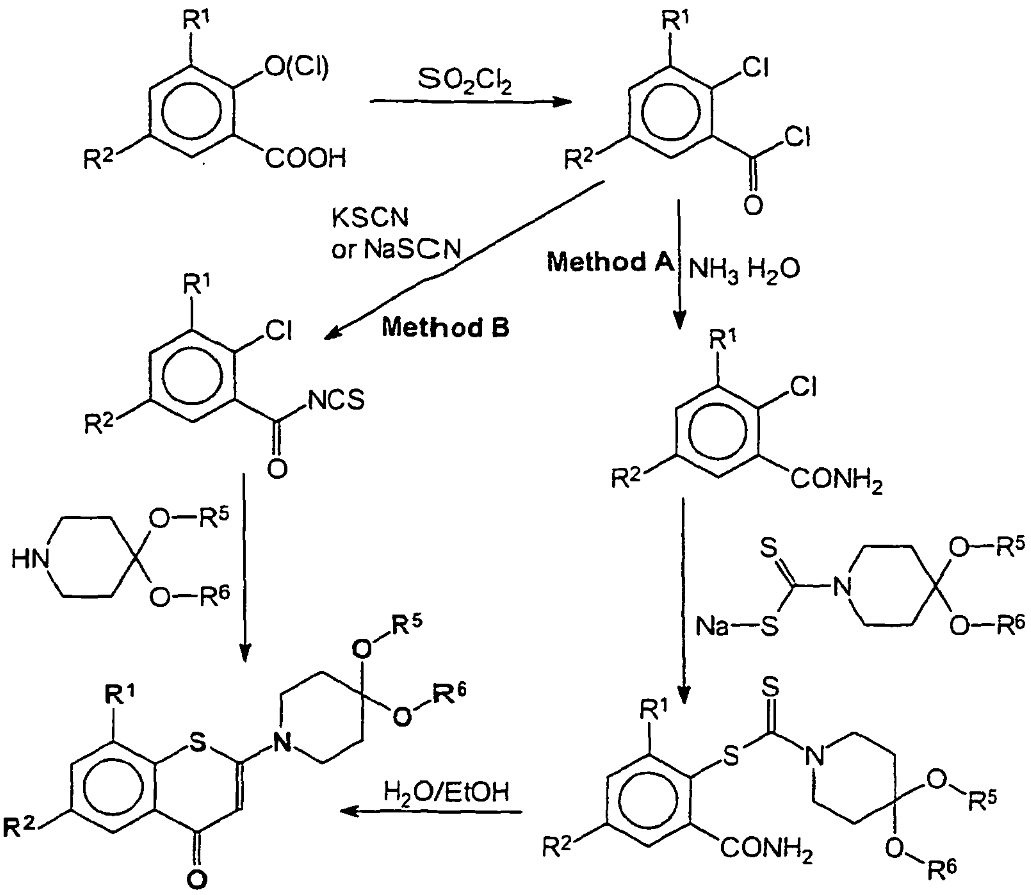


Surprisingly the compound of the invention exhibit strong antibacterial activity, especially against mycobacteria with minimal inhibitory concentrations (MIC) in the range of 0,23pg/ml->10μg/ml for fast growing mycobacteria, of 0,195-1,56 μg/ml for *M. tuberculosis*, including multiresistant strains determined by the classical method and of 0,030μg/ml for *M. tuberculosis* H37Rv determined by the Alamar Blue method. Surprisingly the compound of the invention demonstrates a high level of selectivity for mycobacteria only which reduces the potential for adverse side effects dramatically.
The compound of the invention is non-mutagenic at 5mg/ml in the SOS chromotest.

The compound of the invention is *in vivo* therapeutically active in the murine model of tuberculosis infection superior compared to the main antituberculosis drug isoniazid used as a positive control. 100% of mice survived. All control animals died until day 33.

The compound of the invention, is non toxic. After per os administration of doses ranging up to 2000 mg/kg, the compound was well endured by animals in the first and 24 next coming hours after introducing. During 7 days of investigations the compound did not cause changes in general state and behavior of the mice, it did not affect motor and reflex activity, active and calm cycles, grooming, food consumption, there were no cases of animal death. $LD_{50}$ for compound 2 is > 2000 mg/kg.
Thus, the compound of the invention is useful for the treatment of tubercular infection and other mycobacterial infections, in humans and in animals.

Accordingly, the invention concerns pharmaceutical compositions comprising the compound of the invention or salts thereof.The invention relates furthermore to the compound of the invention or salts thereof for use in a method for the treatment of bacterial infections in mammals.

The compound of the invention is formulated for use by preparing a dilute solution or suspension in pharmaceutically acceptable aqueous, organic or aqueous-organic medium for topical or parenteral administration by intravenous, subcutaneous or intramuscular injection, or for intranasal application; or is prepared in tablet, capsule or aqueous suspension form with conventional excipients for oral administration or as suppositorium.

The compound can be used in dosages from 0,001 - 1.000 mg/kg body weight.

The examples which follow in the subsequent experimental part serve to illustrate the invention but should not be construed as a limitation thereof.

The structures of the compound of the invention were established by modes of synthesis and elementary

analysis, and by nuclear magnetic resonance and/or mass spectra, as well as by X-ray analysis.

Embodiments

Starting materials

Chemicals and solvents were purchased from Lancaster Synthesis (Lancashire, England) or from Aldrich (Sigma-Aldrich Company, St-Louis, US) and were used in the synthesis without additional purification. Melting points were determined according to the BP procedure and are uncorrected (Electrothermal 9001, GB). If analyses are indicated only by the symbols of the elements, analytical results are within ±0.3% of the theoretical values (Carlo-Erba 5500, Italy). NMR spectra were determined with a Varian Unity Plus 400 (USA). Shifts for $^1H$ NMR are reported in ppm downfield from TMS (δ). Mass spectra were obtained using a Finnigan SSQ-700 (USA) instrument with direct inject. Reactions and purity of compounds were controlled by TLC with usage Silicagel 60 $F_{254}$ aluminium sheets (Merck Co, Germany).

Reference Example 1

2-(1,4-dioxa-8-azaspiro[4.5]dec-8-yl)-8-nitro-6-(trifluoromethyl)-1,3-benzothiazin-4-one, (compound 1)

Method A.

To a stirred 50 mL solution of 25% aqueous ammonia was added dropwise a solution of 5 g of 2-chloro-3-nitro-5-trifluoromethylbenzoyl chloride (D.E Welch, R.R.Baron, B.A.Burton, J. Med. Chem. 12; 2; 1969; 299-303) in acetonitrile (10 mL) at -20°C. 10 min later, 50 ml of ethyl acetate was added. The organic phase was separated, washed twice in water, dried over $Na_2SO_4$, treated by activated carbon, filtered and concentrated in vacum. The crude product was purified by crystallization from ethanol. The yield of 2-chloro-3-nitro-5-(trifluoromethyl)benzamide was 92%. mp 195-197°C (methanol).

| | |
|---|---|
| Anal. Calcd. for $C_8H_4ClF_3N_2O_3$: | C, 35.78; H, 1.50; N, 10.43 |
| Found: | C, 36.01; H, 1.53; N, 10.39 |

0.5 g of 2,2-chloro-3-nitro-5-(trifluoromethyl)benzamide was dissolved in a 25 ml of ethanol. The reaction mixture was treated with of 0.5 g of 1,4-dioxa-8-azaspiro[4.5]decane-8-carbodithioic acid sodium salt dihydrate (Z. Ge, R. Li, T. Cheng, Synth. Commun., 29, 18, 1999, 3191 - 3196) and stored for 18 h at room temperature. It was then poured into 50 ml of cooled water and the resulting yellow precipitate was filtered off. Pure final product was obtained after recrystallization twice from ethanol. 2-(Aminocarbonyl)-6-nitro-4-(trifluoromethyl)phenyl-1,4-dioxa-8-azaspiro[4.5]decane-8-carbodithioate is light yellow crystalline solid. Yield 0.47g %. mp 138-140°C.

| | |
|---|---|
| Anal. Calcd. for $C_{11}H_{12}N_4O_2S_2$: | C, 42.57; H, 3.57; N, 9.31; S, 14.21 |
| Found: | C, 42.61; H, 3.67; N, 9.22; S, 14.30 |

0.4 g of 2-(aminocarbonyl)-6-nitro-4-(trifluommethyl)phenyl-1,4-dioxa-8-azaspiro[4.5]decane-8-carbodithioate was dissolved in a 25 ml of ethanol. The reaction mixture was treated with of 0.32 g of $Na_2HPO_4$ x $12H_2O$ and refluxed for 6 h. It was then cooled and ligth yellow precipitate was filtered off and washed by 30 ml methanol. Pure final product was obtained after recrystallization twice from ethanol. 2-(1,4-Dioxa-8-azaspiro[4.5]dec-8-yl)-8-nitro-6-trifluoromethyl)-1,3-benzothiazin-4-oneis light yellow crystalline solid. Yield 0.47g %. mp 211-212°C.
$R_f$ ((hexane-acetone; 2/1) - 0.35
MS m/z 417 ($M^+$).
$^1H$ NOR (DMSO-$d_6$) δ 8.83 and 8.77 (two 1H, two s, 2CH), 3.80 (8H, broad s, $N(CH_2CH_2)_2C$), 2.02 (4H, broad s, $OCH_2CH_2O$) ppm.

| | |
|---|---|
| Anal. Calcd. for $C_{16}H_{14}F_3N_3O_5S$: | C, 46.04; H, 3.38; N, 10.07; S,7.68 |
| Found: | C, 45.94; H, 3.37; N, 10.09; S,7.76 |

Method B. The procedure in detail was the same as described in J.Imrich, P. Kristian, Coll. Czech. Chem. Commun., 47, 1982, 3268-3282; D. Koscik, P. Kristian, J. Gonda, E. Dandarova, Coll. Czech. Chem. Commun., 48, 1983, 3315-3328; D. Koscik, P. Kristian, O. Forgac, Coll. Czech. Chem. Commun., 48, 1983, 3427-3432; T. H. Cronin, H. - J .E. Hess,

Pat. US 3522247. Yield of 2-(1,4-dioxa-8"aza-spiro[4.5]dec-8-yl)-8-mtro-6-trifluoromctbyl)-1,3-benzothiazin-4-one is 0.21%. The compound is identical by spectroscopical data to the compound synthesized by method A.

Example 1

2-(2-methyl-1,4-dioxa-8-azaspiro[4.5]dec-8-yl)-8-nitro-6-(trifluoromethyl)-1,3-benzothiazin-4-one, (compound 2)

Following the procedure of Reference Example 1. Light yellow crystalline solid. Yield 54%. mp 192-3°C.
$R_f$ (hexane-acetone; 2/1) - 0.30.
MS m/z 431 ($M^+$).
$^1$H NMR (DMSO-$d_6$) δ 8.81 and 8.77 (two 1H, two s, 2CH), 4.24 (1H, m, CH), 4.11 (1H, m, CH), 4.06 (4H, broad s, N $(CH_2)_2$), 3,47 (1H, t, CH), 3.27 (1H, s, CH), 1.80 (4H, broad d, $C(CH_2)_2$), 1.23 (3H, d, $CH_3$) ppm.

| | | |
|---|---|---|
| Anal. Calcd. for | $C_{17}H_{16}N_3O_5S$: | C, 47.33; H, 3.74; N, 9.74; S, 7.43 |
| | Found: | C, 47.36; H, 3.80; N, 9.87; S, 7.51 |

Reference Example 2

2-(1,4-dioxa-8-azaspiro[4.5]dec-8-yl)-6,8-dinitro-1,3-benzothiazin-4-one, (compound 3)

Following the procedure of Reference Example 1 with usage of 2-hydroxy-3,5-dinitrobenzoic acid as starting material. Light yellow crystaline solid.
Yield 43%. mp 271-3°C (EtOH/DMF)..
$R_f$ (hexane-acetone; 2/1) - 0.25.
MS m/z 394 ($M^+$).
$^1$H NMR (DMSO-$d_6$) δ 9.15 and 9.12 (two 1H, two s, 2CH), 3.86 (8H, broad s, $N(CH_2CH_2)_2C$), 2.97 (4H, broad s, $OCH_2CH_2O$) ppm.

| | | |
|---|---|---|
| Anal. Calcd. for | $C_{15}H_{14}N_4O_7S$: | C, 45.68; H, 3.58; N, 14.21; S, 8.13 |
| | Found: | C, 45.34; H, 3.56; N, 14.30; S, 7.98 |

Reference Example 3

2-(2-methyl-1,4-dioxa-8-azaspiro[4.5]decyl)-6,8-dinitro-1,3-benzothiazin-4-one, (compound 4)

Following the procedure of Reference Example 1 with usage of 2-hydroxy-3,5-dinitrobenzoic acid as starting material. Yellow crystalline solid. Yield 57%. mp 139-142°C (EtOH/DMF).
$R_f$ (hexane-acetone; 2/1) - 0.50.
MS m/z 408 ($M^+$).
$^1$H NMR (DMSO-$d_6$) δ 9.08 and 9.11 (two 1H, two s, 2CH), 4.23 (1H, m, CH), 4.10 (1H, m, CH), 4.06 (4H, broad s, N $(CH2)_2$), 3,43 (1H, t, CH), 3.27 (1H, s, CH), 1.80 (4H, broad d, $C(CH_2)_2$), 1.20 (3H, d, $CH_3$) ppm.

| | | |
|---|---|---|
| Anal. Calcd. | for $C_{16}H_{16}N_4O_7S$: | C, 47.06; H, 3.95; N, 13.72; S, 7.85 |
| | Found: | C, 46.87; H, 3.91; N, 13.57; S, 7.83 |

Reference Example 4

2-(2,3-dimethyl-1,4-dioxa-8-azaspiro[4.5]dec-8-yl)-8-nitro-6-(trifluoromethyl)-1,3-benzothiazin-4-one, (compound 5)

Following the procedure of Reference Example 1 with usage of 2-hydroxy-3 -nitro-5-trifluoromethylbenzoic acid as starting material. Light yellow crystalline solid. Yield 58%. mp 205-207°C (EtOH/DMF).
$R_f$ (hexane-acetone; 2/1) - 0.55.
MS m/z 44522 ($M^+$).
$^1$H NMR (DMSO-$d_6$) δ 8.82 and 8.77 (two 1H, two s, 2CH), 3.86 (4H, broad c, $N(CH_2)_2$), 3,45-3.53 (2H, m, 2CH), 2.41 (4H, broad d, $C(CH_2)_2$), 1.1.3-1.17 (6H, m, $2CH_3$) ppm.

| | | |
|---|---|---|
| Anal. Calcd. for | $C_{18}H_{18}F_3N_3O_5S$: | C, 48.54; H, 4.07; N, 9.43; S, 7.20 |
| | Found: | C, 48.66; H, 4.12; N, 9.32; S, 7.46 |

Reference Example 5

2-(4,4-diethoxypiperidin-l-yl)-6,8-dinitro-1,3-benzothiazin-4-one, (compound 6)

Following the procedure of Reference Example 1 with usage as starting material 2-hydroxy-3,5-dinitrobenzoic acid. Yellow crystalline solid. Yield 32%. mp 179-181°C (*i*-PrOH).
$R_f$ (hexane-acetone; 2/1) - 0.30,
MS m/z 424 ($M^+$).
$^1H$ NMR (DMSO-$d_6$) δ 9.08 and 9.11 (two 1H, two s, 2CH), 3.60-3.67 (4H, m, $N(CH_2)_2$) 2.11-2.08 (4H, m, $C(CH_2)_2$), 3.47 and 3.57 (two 2H, q, $2OCH_2$), 1.16 (6H, t, $2CH_3$), ppm.

| | | |
|---|---|---|
| Anal. Calcd. | for$C_{17}H_{20}N_4O_7S$: | C, 48.11; H, 4.75; N, 13.20; S, 7.56 |
| | Found: | C, 48.12; H, 4.73; N, 13.41; S, 7.67 |

Reference Example 6

2-(7,12-dioxa-3-azaspiro[5.6]dodec-3-yl)-6,8-dinitro-1,3-benzothiazin-4-one, (compound 7)

Following the procedure of Reference Example 1 with usage as starting material 2-hydroxy-3,5-dinitrobenzoic acid. Yellow crystalline solid. Yield 51%. mp 193-195°C (*i*-PrOH/DMF).
$R_f$ (hexane-acetone; 2/1) - 0.45.
MS m/z 422 ($M^+$).
$^1H$ NMR (DMSO-$d_6$) δ 8.97 and 9.16 (two 1H, two s, 2CH), 3.57-3.74 (8H, m, $4CH_2$), 1.93-2.35 (8H, m, $4CH_2$)ppm.
Anal. Calcd. for $C_{17}H_{18}N_4O_7S$: C, 48.34; H, 4.30; N, 13.26; S, 7.56
Found: C, 48.21; H, 4.43; N, 13.30; S, 7.66

Reference Example 7

2-(1,4-dioxa-8-azaspiro[4.5]dec-8-yl)-7-methyl-6,8-dinitro-1,3-benzothiazin-4-one, (compound 8)

Following the procedure of Reference Example 1 with usage as starting material 2-hydroxy-4-methyl-3,5-dinitrobenzoic acid. Yellow crystalline solid.
Yield 51%. mp 207-210°C (*i*-PrOH/DMF).
$R_f$ (hexane-acetone; 2/1) - 0.30.
MS m/z 408 ($M^+$).
$^1H$ NMR (DMSO-$d_6$) δ 8.77 (1H, s, CH), 3.86 (8H, broad s, $N(CH_2CH_2)_2C$), 2.97 (4H, broad c, $OCH_2CH_2O$), 2.79 (3H, s, $CH_3$) ppm. ppm.

| | | |
|---|---|---|
| Anal. Calcd. for | $C_{16}H_{16}N_4O_7S$: | C, 47.06; H, 3.95; N, 13.72; S,7.85 |
| | Found: | C, 47.12; H, 4.01; N, 13.69; S,7.94 |

Reference Example 8

2-(1,4-dioxa-8-azaspiro[4.5]dec-8-yl)-8-nitro-4-oxo-1,3-benzothiazine-6-carbonitrile, (compound 9)

To a stirred solution of 5 g (19 mmol) 2-hydroxy-5-iodobenzoic acid in 50ml DMF was added by small portions dry 2.5 g (22 mmol) of CuCN (I). The reaction mixture was refluxed during 5 h, 100 ml of water and 50 ml ethylacetate were added. After it cone. Hydrochloric acid was added up to pH ~ 3 very carefully under good ventilation. The organic phase was separated, washed twice in water, dried over $Na_2SO_4$, treated by activated carbon, filtered and concentrated in vacum. The crude product was purified by crystallization from water. The yield of 5-cyano-2-hydroxybenzoic acid was 71%. Following the procedure of Example 1. Yield 44%. mp 217-220°C (EtOH/DMF).
$R_f$ (hexane-acetone; 2/1) - 0.50.

MS m/z 374 ($M^+$).
$^1H$ NMR (DMSO-$d_6$) δ 8.74 and 8.67 (two 1H, two s, 2CH), 3.41 (8H, broad s, $N(CH_2CH_2)_2C$), 2.93 (4H, broad s, $OCH_2CH_2O$) ppm.

| | | |
|---|---|---|
| Anal. Calcd. for | $C_{16}H_{14}N_4O_5S$: | C, 51.33; H, 3.77; N, 14.97; S,8.57 |
| | Found: | C, 51.30; H, 3.84; N, 14.89;S, 8.62 |

Reference Example 9

2-(2-methyl-1,4-dioxa-8-azaspiro[4.5]dec-8-yl)-8-nitro-4-oxo-1,3-benzothiazine-6-carbonitrile, (compound 10)

Following the procedure of Reference Example 8. Yellow crystalline solid. Yield 34%. mp 251-253°C (EtOH/DMF).
$R_f$ (hexane-acetone; 2/1) - 0.40.
MS m/z 388 ($M^+$).
$^1H$ NMR (DMSO-$d_6$) δ 8.73 and 8.61 (two 1H, two s, 2CH), 4.23 (1H, m, CH), 4.11 (1H, m, CH), 4.07 (4H, broad s, N $(CH_2)_2$), 3,51 (1H, t, CH), 3.27 (1H, s, CH), 1.81 (4H, broad d, $C(CH_2)_2$), 1.22 (3H, d, $CH_3$) ppm ppm.

| | | |
|---|---|---|
| Anal. Calcd. for | $C_{17}H_{16}N_4O_5S$: | C, 52.57; H, 4.15; N, 14.43; S,8.26 |
| | Found: | C, 52.42; H, 4.08; N, 1.4.50; S,8.27 |

Reference Example 10

2-(1,5-dioxa-9-azaspiro[5.5]undec-9-yl)-8-nitro-4-oxo-1,3-benzothiazine-6-carbontrile, (compound 11)

Following the procedure of Reference Example 8. Yellow crystalline solid. Yield 40%. mp 230-232°C (EtOH/DMF).
$R_f$ (hexane-acetone; 2/1) - 0.15.
MS m/z 388 ($M^+$).
$^1H$ NMR (DMSO-$d_6$) δ 8.74 and 8.61 (two 1H, two s, 2CH), 3.29-3.65 (6H, m, $3CH_2$), 2.38 (4H, broad s, $2CH_2$), 1.82-1.93 (4H, m, $2CH_2$) ppm.

| | | |
|---|---|---|
| Anal. Calcd. for | $C_{17}H_{16}N_4O_5S$: | C, 52.57; H, 4.15; N, 14.43; S,8.26 |
| | Found: | C, 52.52; H, 4.11; N, 14.59; S, 8.13 |

Example 2

Determination of the *in vitro* inhibitory activity of the compound of the invention against mycobacteria.

The antibacterial activities of the compound against *Mycobacterium smegmatis* SG 987, *M aureum* SB66*, M. vaccae* IMET 10 10670 and *M. fortuitum* B were tested by determination of minimal inhibitory concentrations (MIC) by the micro broth dilution method in Mueller-Hinton broth (Difco) according to the NCCLS guidelines [National Committee for Clinical Laboratory Standards: Methods for dilution antimicrobial susceptibility tests for bacteria that grow aerobically; 5th Ed.; Villanova, Ed.; Approved standard Document M7-A5. NCCLS, (2000)]

Activity against *M. tuberculosis* H37Rv was tested by the following method for determination of minimal inhibitory concentrations (MIC) and minimal bactericidal concentrations (MBC):

Strains were inoculated onto solid Lowenstein-Jensen medium. After 21 days, the cultures grown were used to prepare an inoculum suspension corresponding to 5 x $10^8$ microbial cells/ml). With 0,2 ml of that suspension tubes with 2 ml liquid Shkolnikova medium, containing corresponding concentrations of compounds under study- from 100,0 to 0,195 Mg/ml, were inoculated. After 14 days of incubation at 37 °C the tubes with liquid medium were centrifuged for 15min. at 3000 RPM. After discarding the supernatant, the sediment was resuspended in 0,8 ml of sterile 0,9% NaCl. 0,1 ml of the suspension was used to prepare smears subsequently stained by the Ziehl-Neelsen method. The remaining sediment was inoculated in 0,2 ml volumes into three tubes with solid drug free Lowenstein-Jensen medium to determine minimal bactericidal concentrations (MBC). The results were read after 21-28 days of cultivation at 37 °C. Controls were tubes cultured with test-strains not treated with the studied agents.

Minimal bactericidal concentration of drugs (MBC) was considered as the drug concentration completely inhibiting the growth of mycobacteria on the solid medium. The bacteriostatic effect (MIC) was characterized by the

presence of only individual mycobacteria in the smear and a strong decrease in the number of colonies grown on solid media compared to the controls.

The results are presented in Tables 1 and 2.

Table 1: Antimicrobial activity of compounds determined by minimal inhibitory concentrations MIC [μg/ml]

| Compound. | M. smegmatis | M.vaccae | M.fortuitum |
|---|---|---|---|
| 1 | 12,5 n g/ml | 3,12ng/ml | 12,5 ng/ml |
| 2 | 1,56 ng/ml | 0,76pg/ml | 0,023 pg/ml |
| 3 | 0,2,μg/ml | 0,0015 μg/ml | 0,006 μg/ml |
| 4 | 0,2 μg/ml | 0,003 μg/ml | 0,003 μg/ml |
| 5 | 6,25ng/ml | 0,078ng/ml | 0,078ng/ml |
| 6 | >10 μg/ml | 0,04 μg/ml | 0,08 μg/ml |
| 7 | 0,78 μg/ml | 0,003 μg/ml | 0,003 μg/ml |
| 8 | 0,4 μg/ml | 0,025 μg/ml | 0,025 μg/ml |
| 9 | 0,05 μg/ml | 3,12 ng/ml | 25 ng/ml |
| 10 | 25 ng/ml | 3,12 ng/ml | 12,5 ng/ml |
| 11 | 0,05 μg/ml | 6,25 ng/ml | 25 ng/ml |

Table 2: Antimicrobial activity of compounds against *Mycobacterium tuberculosis* H37Rv and clinical isolates 6341 and 6374 as determined by minimal inhibitory concentrations (MIC) and minimal bactericidal concentrations (MBC)

| Strain | Compound | MBC (μg/mL) | MIC (μg/mL) | MBC (μg/mL) mean | MIC (μg/mL) mean |
|---|---|---|---|---|---|
| H37Rv | 10 | 0,58 | 0,39 | 0,71 | 0,45 |
| 6341 | | 0,78 | 0,58 | | |
| 6374 | | 0,78 | 0,39 | | |
| H37Rv | 9 | 0.29 | 0,195 | 0,75 | 0,52 |
| 6341 | | 1,17 | 0,78 | | |
| 6374 | | 0,78 | 0,58 | | |
| H37Rv | 2 | 0,58 | 0,39 | 0,45 | 0,29 |
| 6341 | | 0.39 | 0,29 | | |
| 6374 | | 0,39 | 0,195 | | |
| H37Rv | 5 | 0,58 | 0,39 | 0,45 | 0,39 |
| 6341 | | 0,39 | <0,39 | | |
| 6374 | | 0,39 | <0,39 | | |
| H37Rv | 1 | 0,58 | 0,39 | 1,75 | 1,17 |
| 6341 | | 2,34 | 1,56 | | |
| 6374 | | 2,34 | 1,56 | | |
| H37Rv | Isoniazid (INH) | 1,15 | 0,97 | 1,15 | 0,97 |
| 6341 | | >100 | >100 | Not active, >100 | |
| 6374 | | >100 | >100 | Not active, >100 | |

Example 3

Determination of the *in vivo* inhibitory activity of the compounds of the invention against *Mycobacterium tuberculosis* in the murine TB model To determine the chemotherapeutic efficacy we used BALB/c line mice with experimental hematogenously disseminated tuberculosis. The mice were obtained from the Central Animal Nursery of the Russian Academy of Medical Sciences. In this study we included mice after quarantine, standardized by weight (20-25 g) and male only. The mice were infected with a 2-week virulent culture of *Mycobacterium tuberculosis* H37Rv by intravenous injection (into tail vein) of the mycobacterial suspension at a dose of 5 x $10^6$ CFU (Colony Forming Unit) in 0,5 ml saline. All the experimental animals were divided into groups depending on the treatment regimen used (Table 3). Tested drug doses were selected based on the data from literature and on results of previous investigations.

Table 3:

| № group | Compound | Dose (mg/kg) | Number of animals per group |
|---|---|---|---|
| 3 | 2 | 12 | 10 |
| 4 | 2 | 25 | 10 |
| 5 | Isoniazid (INH) | 25 | 10 |
| 6 | without treatment | | 10 |

Treatment was started the next day after infection. The drugs were introduced orally as suspension in carboxymethylcellulose/water with a small quantity PEG-400.
Chemotherapy was administered daily 6 times per week (except Sunday).
The animals were killed with ether narcosis. To determine the efficacy of each treatment regimen we registered macroscopical changes in parenchymal organs of the mice, growth of mycobacteria from pathologic material on solid media, as well as a bacterioscopical index of organ injury. We carried out a qualitative and quantitative analysis of macroscopical changes in the liver, spleen and lungs and calculated an injury index (using a four-score scale).
Macroscopical evaluation of the efficacy of each treatment regimen was expressed in the efficacy index, calculated using a formula.

$$\text{Efficacy index} = 100\% - \frac{\text{Injury index of the studied group}}{\text{Injury index of the control group}} \times 100$$

Microbiological examination included culture for determination of CFU in parenchymal organs. For this purpose, we homogenisated the right lung and separately the spleen with 6% sulfuric acid, centrifuged, washed by water and saline. The yield (about 0,5 mL) was diluted by 1,0 mL of saline and homogenisated. This suspension (0,5 mL) of test organs was diluted 100 and 1000 times by saline and was distributed on solid Finn-2 medium. The cultures were incubated at 37°C for 1 months and read weekly starting from day 10. After 28 days CFU's were counted.
Data of macroscopical and microbiological examinations of parenchymal organs of mice which died during the experiment were also considered in the overall assessment of the experimental results which are represented in tables 4-6.

Table 4: Indexes of origan injury in mice and treatment efficacy

| Group | Drug | Dose (mg/kg) | Injury index | Efficacy index (%) |
|---|---|---|---|---|
| 3 | Compound 2 | 12 | 2,1 | 44,7 |
| 4 | Compound 2 | 25 | 1,0 | 78 |

(continued)

| Group | Drug | Dose (mg/kg) | Injury index | Efficacy index (%) |
|---|---|---|---|---|
| 5 | INH, Isoniazid | 25 | 1,2 | 70,5 |
| 6 | Control | -- | 3,8 | -- |

Table 5: Results of microbiological examination of right lung and spleen of experimental mice (42 days after inoculation of the culture medium)

| Group | Compound | Dose (mg/kg) | right lung Culture, without dilution CFU | spleen Culture, without dilution CFU |
|---|---|---|---|---|
| 3 | 2 | 12 | ~ 60 | ~ 60 |
| 4 | 2 | 25 | ~ 35 | ~ 35 |
| 5 | INH, Isoniazid | 25 | ~ 40 | ~ 40 |
| 6 | Control | -- | > 120 (total growth) | > 120 (total growth) |

Table 6: Survival of animals

| Day of Treatment | Group 3 Compound 2 | Group 4 Compound 2 | Group 5 INH | Group 6 Control |
|---|---|---|---|---|
| 1 | 10 | 10 | 10 | 10 |
| 2 | 10 | 10 | 10 | 10 |
| 3 | 10 | 10 | 10 | 10 |
| 4 | 10 | 10 | 10 | 10 |
| 5 | 10 | 10 | 10 | 10 |
| 6 | 10 | 10 | 10 | 10 |
| 7 | 10 | 10 | 10 | 10 |
| 8 | 10 | 10 | 10 | 10 |
| 9 | 10 | 10 | 10 | 10 |
| 10 | 10 | 10 | 10 | 10 |
| 11 | 10 | 10 | 10 | 10 |
| 12 | 10 | 10 | 10 | 10 |
| 13 | 10 | 10 | 10 | 9 |
| 14 | 10 | 10 | 10 | 9 |
| 15 | 10 | 10 | 10 | 9 |
| 16 | 10 | 10 | 10 | 9 |
| 17 | 10 | 10 | 10 | 9 |
| 18 | 10 | 10 | 10 | 9 |
| 19 | 10 | 10 | 10 | 9 |
| 20 | 10 | 10 | 10 | 8 |
| 21 | 10 | 10 | 10 | 8 |

(continued)

| Day of Treatment | Group 3 Compound 2 | Group 4 Compound 2 | Group 5 INH | Group 6 Control |
|---|---|---|---|---|
| 22 | 10 | 10 | 10 | 8 |
| 23 | 10 | 10 | 10 | 8 |
| 24 | 10 | 10 | 10 | 8 |
| 25 | 10 | 10 | 10 | 5 |
| 26 | 10 | 10 | 10 | 4 |
| 27 | 100% | 100% | 100% | 40% |
| All control animals died until day 33 | | | | |

## Claims

1. 2-(2-methyl-1,4-dioxa-8-azaspiro[4.5]dec-8-yl)-8-nitro-6-(trifluoromethyl)-1,3-benzothiazin-4-one.

2. Salts of 2-(2-methyl-1,4-dioxa-8-azaspiro[4.5]dec-8-yl)-8-nitro-6-(trifluoromethyl)-1,3-benzothiazin-4-one.

3. A pharmaceutical composition comprising a compound according to any of claims 1 or 2.

4. A compound according to any of claims 1 or 2 for use in a method for the therapeutic treatment of tuberculosis infection or leprosy infection in mammals.

## Patentansprüche

1. 2-(2-Methyl-1,4-dioxa-8-azaspiro[4.5]dec-8-yl)-8-nitro-6-(trifluormethyl)-1,3-benzothiazin-4-on.

2. Salze von 2-(2-Methyl-1,4-dioxa-8-azaspiro[4.5]dec-8-yl)-8-nitro-6-(trifluormethyl)-1,3-benzothiazin-4-on.

3. Pharmazeutische Zusammensetzung, umfassend eine Verbindung nach einem der Ansprüche 1 oder 2.

4. Verbindung nach einem der Ansprüche 1 oder 2 zur Anwendung bei einem Verfahren zur therapeutischen Behandlung von Tuberkulose-Infektionen oder Lepra-Infektionen bei Säugetieren.

## Revendications

1. 2-(2-méthyl-1,4-dioxa-8-azaspiro[4.5]déc-8-yl)-8-nitro-6-(trifluorométhyl)-1,3-benzothiazin-4-one.

2. Sels de 2-(2-méthyl-1,4-dioxa-8-azaspiro[4.5]déc-8-yl)-8-nitro-6-(trifluorométhyl)-1,3-benzothiazin-4-one.

3. Composition pharmaceutique comprenant un composé selon l'une quelconque des revendications 1 ou 2.

4. Composé selon l'une quelconque des revendications 1 ou 2 destiné à une utilisation dans un procédé pour le traitement thérapeutique d'une infection tuberculeuse ou d'une infection lépreuse chez des mammifères.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- AR 242567 A1
- AU 3704400 A1
- CA 1322551 C1
- EP 0245901 B1
- US 3522247 A, T. H. Cronin, H. - J .E. Hess


### Non-patent literature cited in the description


- **B.R.BLOOM ; J.L.MURRAY.** tuberculosis: commentary on a reemergent killer. *Science,* 1992, vol. 257, 1055-1064
- **D.E WELCH ; R.R.BARON ; B.A.BURTON.** *J. Med. Chem.,* 1999, vol. 12 (2), 299-303
- **Z. GE ; R. LI ; T. CHENG.** *Synth. Commun.,* 1999, vol. 29 (18), 3191-3196
- **J.IMRICH ; P. KRISTIAN.** *Coll. Czech. Chem. Commun.,* 1982, vol. 47, 3268-3282
- **D. KOSCIK ; P. KRISTIAN ; J. GONDA ; E. DANDAROVA.** *Coll. Czech. Chem. Commun.,* 1983, vol. 48, 3315-3328
- **D. KOSCIK ; P. KRISTIAN ; O. FORGAC.** *Coll. Czech. Chem. Commun.,* 1983, vol. 48, 3427-3432
- NCCLS guidelines